# EUROPEAN PATENT APPLICATION

(11) **EP 4 846 980 A1**
(43) Date of publication of application: **02.12.2026**
(21) Application number: 26180687.1
(22) Date of filing: 22.05.2026
(51) Int. Cl.: A61F 2/24

(54) **EART VALVE REPAIR DEVICES**

(30) Priority: 27.05.2025 US 202563812665 P; 07.05.2026 US 202619670095
(71) Applicant: Medtronic, Inc., Minneapolis, Minnesota 55432 (US)
(72) Inventor: Duffy, Niall F., Galway (IE)
(74) Representative: Zimmermann & Partner Patentanwälte mbB

(57) **Abstract**

A heart valve repair device having a delivery configuration and a deployed configuration includes a first anchor, a second anchor, and a tension member. With the heart valve repair device in the delivery configuration, the first anchor is spaced a first longitudinal distance from the second anchor. The tension member is configured to transition the heart valve repair device to the deployed configuration by translating the second anchor relative to the first anchor such that the first anchor is spaced a second longitudinal distance from the second anchor, the second longitudinal distance being shorter than the first longitudinal distance.

## Description

### CROSS-REFERENCE TO RELATED APPLICATIONS

This application claims the benefit of the filing date of U.S. Provisional Application No. 63/812,665, filed May 27, 2025, and U.S. Patent Application No. 19/670,095, filed May 7, 2026, the contents of which are incorporated by reference herein in their entirety

### FIELD

The present technology is related generally to devices and methods for repair of heart valves, and more particularly to devices and methods for use in repair of the mitral valve.

### BACKGROUND

The human heart is a four-chambered, muscular organ that provides blood circulation through the body during a cardiac cycle. The four main chambers include the right atrium and right ventricle which supplies the pulmonary circulation, and the left atrium and left ventricle which supplies oxygenated blood received from the lungs to the remaining body. To ensure that blood flows in one direction through the heart, atrioventricular valves (tricuspid and mitral valves) are present between the junctions of the atria and the ventricles, and semi-lunar valves (pulmonary valve and aortic valve) govern the exits of the ventricles leading to the lungs and the rest of the body, respectively. These valves contain leaflets or cusps that open and shut in response to blood pressure changes caused by the contraction and relaxation of the heart chambers. The leaflets move apart from each other to open and allow blood to flow downstream of the valve, and coapt to close and prevent backflow or regurgitation in an upstream manner.

Diseases associated with heart valves, such as those caused by damage or a defect, can include stenosis and valvular insufficiency or regurgitation. For example, valvular stenosis causes the valve to become narrowed and hardened, which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots, which can increase the likelihood of stroke or pulmonary embolism. Symptoms can often be severe enough to be debilitating and/or life threatening.

In some instances, such as with a native mitral valve, the leaflets of the native valve do not close due to damaged or loosened chordae, which couple the native mitral valve leaflets to the left ventricle.

### BRIEF SUMMARY

The techniques and devices of this disclosure generally relate to devices and methods for repairing a heart valve.

In an example hereof, the present disclosure relates to a heart valve repair device having a delivery configuration and a deployed configuration. The heart valve repair device comprises: a first anchor having an open state and a closed state; a second anchor having an open state and a closed state; and a tension member coupled to the first anchor and the second anchor. The first anchor is spaced a first longitudinal distance from the second anchor when the heart valve repair device is in the delivery configuration. The tension member is configured to translate the second anchor relative to the first anchor such that the first anchor is spaced a second longitudinal distance from the second anchor, the second longitudinal distance being shorter than the first longitudinal distance.

In another example hereof, in the heart valve repair device of any of the preceding or following examples, the first anchor includes a first body portion and a first claw portion. The first anchor in the open state is configured to receive a first portion of a chordae therein and the first anchor in the closed state is configured to grasp the first portion of the chordae.

In another example hereof, in the heart valve repair device of any of the preceding or following examples, the second anchor includes a second body portion and a second claw portion. The second anchor in the open state is configured to receive a second portion of the chordae and the second anchor in the closed state is configured to grasp the second portion of the chordae.

In another example hereof, in the heart valve repair device of any of the preceding or following examples, the tension member is configured to translate the second anchor relative to the first anchor with the chordae grasped by the first anchor and the second anchor such that the chordae is shortened.

In another example hereof, in the heart valve repair device of any of the preceding or following examples, the first anchor and the second anchor are shape-set to the closed state.

In another example hereof, in the heart valve repair device of any of the preceding or following examples, the tension member comprises a suture slightly coupled to the first anchor and fixedly secured to the second anchor such that pulling the tension member pulls the second anchor towards the first anchor.

In another example hereof, in the heart valve repair device of any of the preceding or following examples, the heart valve repair device further comprises a lock configured to secure the tension member to the first anchor with the heart valve repair device in the deployed configuration such that the first anchor is spaced the second longitudinal distance from the second anchor.

In another example hereof, in the heart valve repair device of any of the preceding or following examples, the tension member includes a first end fixedly secured to the first anchor and a second end fixedly secured to the second anchor. The tension member is shape-set such that the first anchor is spaced the second longitudinal distance from the second anchor, and when in the delivery configuration the tension member is placed in tension such that the first anchor is spaced the first longitudinal distance from the second anchor.

In another example hereof, in the heart valve repair device of any of the preceding or following examples, the tension member is a spring.

In another example hereof, in the heart valve repair device of any of the preceding or following examples, the tension member is a shape-set V-shaped connector.

In another example hereof, the present disclosure relates to a method of repairing a native heart valve comprising: delivering a heart valve repair device to a site of the native heart valve, wherein the heart valve repair device comprises a first anchor, a second anchor, and a tension member, wherein the heart valve repair device is delivered to the site in a delivery configuration; grasping a first portion of chordae with the first anchor; grasping a second portion of the chordae with the second anchor, wherein the first anchor is spaced a first longitudinal distance from the second anchor; and with the first portion and the second portion of the chordae grasped by the first anchor and the second anchor, respectively, transitioning the tension member to a deployed state such that the first anchor is spaced a second longitudinal distance from the second anchor, wherein the second longitudinal distance is shorter than the first longitudinal distance, thereby shortening or tightening the chordae.

In another example hereof, in the method of any of the preceding or following examples, grasping the first portion of the chordae with the first anchor comprises: delivering the first anchor to a position adjacent the first portion of the chordae in a closed state of the first anchor; transitioning the first anchor to an open state; receiving the first portion of the chordae in the first anchor with the first anchor in the open state; and transitioning the first anchor to a closed state to grasp the first portion of the chordae.

In another example hereof, in the method of any of the preceding or following examples, grasping the second portion of the chordae with the second anchor comprises: delivering the second anchor to a position adjacent the second portion of the chordae in a closed state of the second anchor; transitioning the second anchor to an open state; receiving the second portion of the chordae in the second anchor with the second anchor in the open state; and transitioning the second anchor to a closed state to grasp the second portion of the chordae.

In another example hereof, in the method of any of the preceding or following examples, the tension member is a suture slidably coupled to the first anchor and fixed coupled to the second anchor, wherein transitioning the tension member to the deployed state comprises pulling the tension member such that the second anchor is pulled towards the first anchor.

In another example hereof, in the method of any of the preceding or following examples, further comprising deploying a lock to lock the tension member in the deployed state.

In another example hereof, in the method of any of the preceding or following examples, further comprising after transitioning the tension member to the deployed state, severing the tension member.

In another example hereof, in the method of any of the preceding or following examples, the tension member includes a first end fixedly secured to the first anchor and a second end fixedly secured to the second anchor. In the delivery configuration, the tension member is tensioned such that the first anchor is spaced the first longitudinal distance from the second anchor, and the tension member is shape-set to the deployed state such that transitioning the tension member to the deployed state comprises releasing the tension member such that the tension member returns to the deployed state.

In another example hereof, in the method of any of the preceding or following examples, the tension member is a spring.

In another example hereof, in the method of any of the preceding or following examples, the tension member is a shape-set V-shaped connector.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings are not to scale.
FIG. 1 depicts a schematic illustration of a heart having native valve structures.
FIG. 2 depicts a schematic sectional illustration of a left ventricle of a heart showing anatomical structures and a native mitral valve.
FIG. 3 depicts a perspective view of a heart valve repair device in a deployed configuration according to embodiments hereof.
FIG. 4 depicts a perspective view of the heart valve repair device in a delivery configuration according to embodiments hereof.
FIG. 5 depicts a perspective view of an anchor member of the heart valve repair device of FIG. 3 in a closed state according to embodiments hereof.
FIG. 6 depicts a perspective view of the anchor member of the heart valve repair device of FIG. 3 in an open state according to embodiments hereof.
FIG. 7 depicts a perspective view of a lock of the repair device of FIG. 3 according to embodiments hereof.
FIG. 8 depicts a side view of a delivery catheter according to embodiments hereof.
FIG. 9 depicts a side view of the delivery catheter of FIG. 8 with a first and a second balloon each in an inflated state according to embodiments hereof.
FIG. 10 depicts a cross-sectional view taken at line A-A of FIG. 8, according to embodiments hereof.
FIG. 11 depicts a side-cross-sectional view of the delivery catheter of FIG. 8, according to embodiments hereof.
FIG. 12 depicts a perspective view of a heart valve repair device in a deployed configuration according to embodiments hereof.
FIG. 13 depicts a perspective view of the heart valve repair device of FIG. 12 in a delivery configuration according to embodiments hereof.
FIG. 14 depicts a perspective view of a heart valve repair device in a deployed configuration according to embodiments hereof.
FIG. 15 depicts a perspective view of the heart valve repair device of FIG. 14 in a delivery configuration according to embodiments hereof.
FIG. 16 is a flow chart depicting a method for delivering and deploying the heart valve repair device of FIG. 3, according to embodiments hereof.
FIGS. 17-23 depict illustrations of several of the steps of the method of FIG. 16 for delivering and deploying the heart valve repair device of FIG. 3.

### DETAILED DESCRIPTION OF THE INVENTION

Specific embodiments of the present disclosure are now described with reference to the figures wherein like reference numbers indicate identical or functionally similar elements. The following detailed description describes examples of embodiments of the invention and is not intended to limit the present technology or the application and uses of the present technology. Although the description of embodiments hereof is in the context of a transcatheter heart valve repair device for use at a native mitral valve, the present technology may also be used for procedures associated with other native heart valves. Furthermore, there is no intention to be bound by any expressed or implied theory presented in the preceding Field, Background, Brief Summary, or the following Detailed Description.

The terms "distal" and "proximal," when used in the following description to refer to a native vessel, native valve, or implanted medical device are with reference to the direction of blood flow. Thus, "distal" and "distally" refer to positions in a downstream direction with respect to the direction of blood flow, and the terms "proximal" and "proximally" refer to positions in an upstream direction with respect to the direction of blood flow. When used in the following description to refer to a catheter or similar device "distal" and "proximal" are with respect to the location of an operator using the device or the handle of such a device. Thus, distal" and "distally" refer to positions in a direction away from the operator or handle end of the device, and the terms "proximal" and "proximally" refer to positions in a direction toward the operator or handle end of the device.

The terms "closed" and "open," when used in the following description to refer to an anchor of the repair device are with reference to the circumferential completeness of a wall defining a lumen or lumens of the anchor. Thus, "closed" refer to an anchor with a continuous circumferential inner surface defining the lumen or lumens, and the term "open" refers to an anchor wherein the inner surface defining the lumen is not circumferentially continuous.

In addition, the term "shape memory" is used in the following description with reference to one or more tension members is intended to convey that the tension members are shaped or formed from a material that can be provided with a mechanical memory to return the structure from an altered shape to its original shape. Non-exhaustive examples of shape memory materials include a pseudo-elastic metal such as a nickel titanium alloy or nitinol, various shape memory polymers, or a so-called super alloy, which may have a base metal of nickel, cobalt, chromium, or other metal. Mechanical memory may be imparted to a tension members by thermal treatment to achieve a spring temper in stainless steel, for example, or to set a shape memory in a susceptible metal alloy, such as nitinol.

Embodiments disclosed herein are directed to heart valve repair devices, hereafter referred to as a "repair device," that include a first anchor, a second anchor, and a tension member deployed at opposite ends on a native chordae tendineae, hereafter referred to as a "chordae". The repair devices are configured to tighten chordae to bring the native valve leaflets closer together to reduce valve regurgitation.

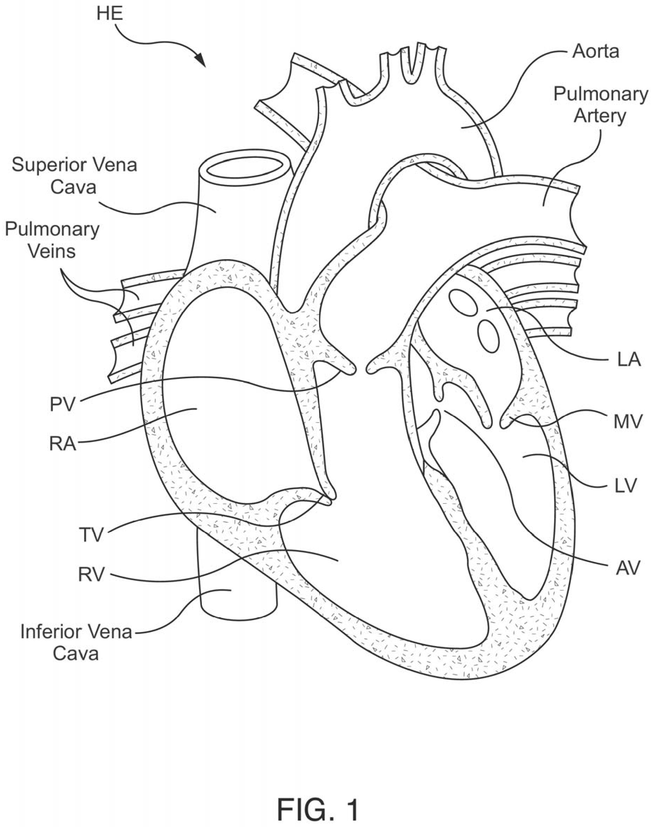

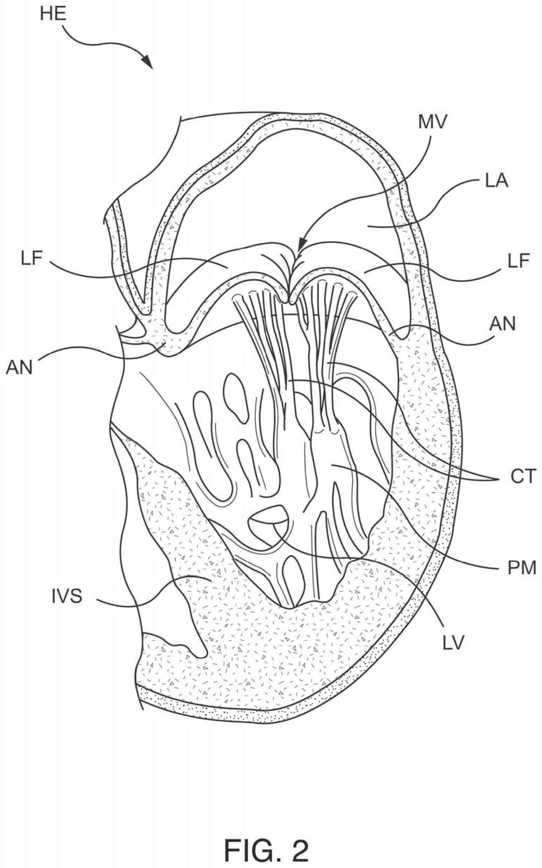

FIG. 1 is a schematic sectional illustration of a heart HE that depicts the four heart chambers (right atrium RA, right ventricle RV, left atrium LA, left ventricle LV) and native valve structures (tricuspid valve TV, mitral valve MV, pulmonary valve PV, aortic valve AV). FIG. 2 is a schematic sectional illustration of a left ventricle LV and left atrium LA of a heart HE showing anatomical structures and a native mitral valve MV. Referring to FIGS. 1 and 2, the heart HE comprises the left atrium LA that receives oxygenated blood from the lungs via the pulmonary veins. The left atrium LA pumps the oxygenated blood through the mitral valve MV and into the left ventricle LV during ventricular diastole. The left ventricle LV contracts during systole and blood flows outwardly through the aortic valve AV, into the aorta into the remainder of the body.

In a healthy heart, the valve leaflets LF of the mitral valve MV meet evenly, or coapt, to close and prevent backflow of blood during contractions of the left ventricle LV. Referring to FIG. 2, the valve leaflets LF attach the surrounding heart structure via a dense, fibrous ring of connective tissue, called an annulus AN which is distinct from both the leaflet tissue LF as well as the adjoining muscular tissue of the heart wall. The flexible leaflet tissue of the mitral valve leaflets LF are connected to papillary muscles PM, which extend upwardly from the lower wall of the left ventricle LV, and the inter-ventricular septum IVS, via branching tendons called chordae tendineae CT. In a heart HE having a mitral valve MV in which the valve leaflets LF do not sufficiently coapt or meet, leakage from the left ventricle LV into the left atrium LA may occur. Several structural defects can cause the mitral valve leaflets LF to inadequately coapt in this manner, and subsequent regurgitation to occur, including ruptured chordae tendineae CT, impairment of papillary muscles PM, e.g., due to his ischemic heart disease, and enlargement of the heart, and/or mitral valve annulus AN, e.g., cardiomyopathy and damaged tissue cords, e.g., damaged chordae.

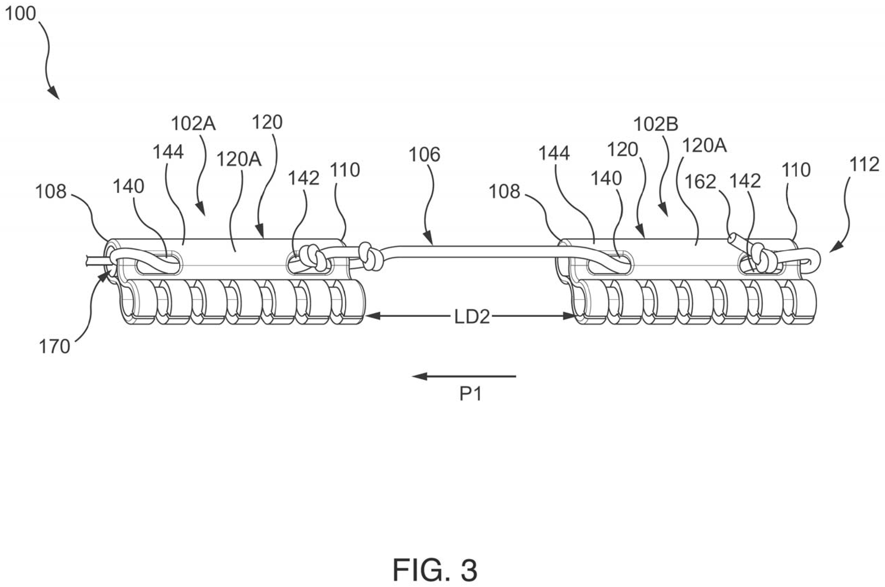

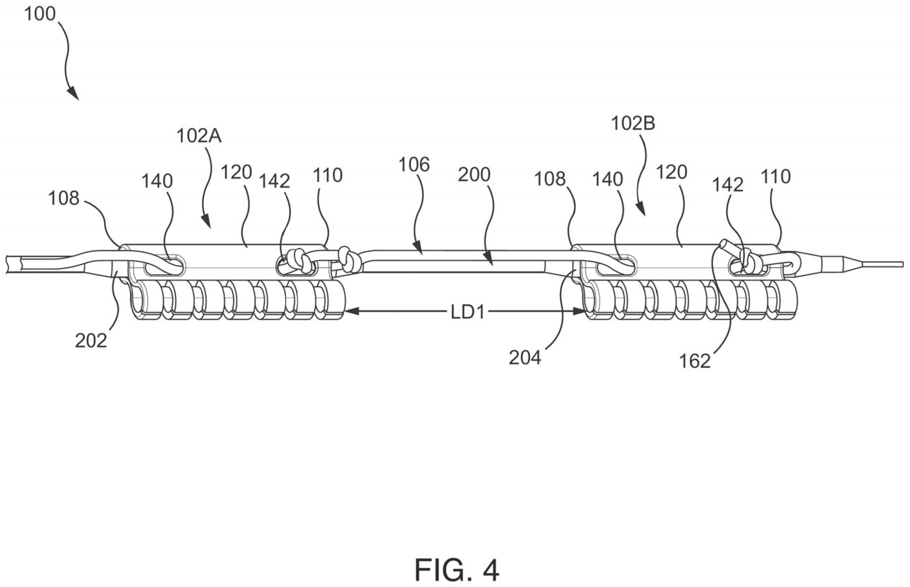

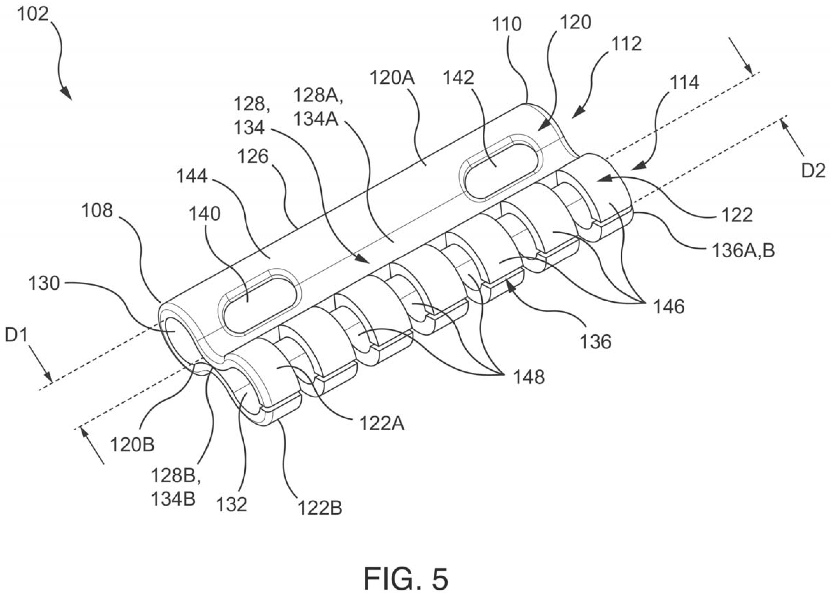

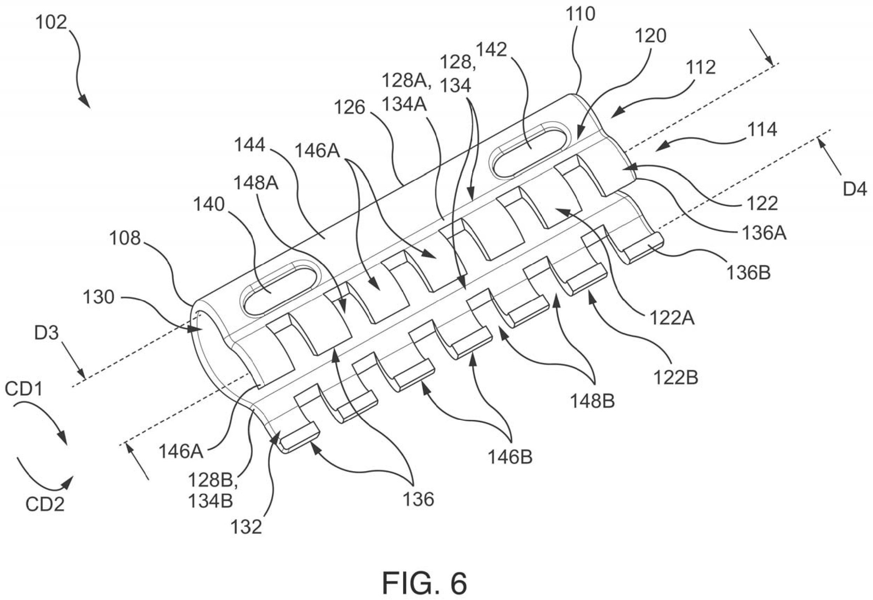

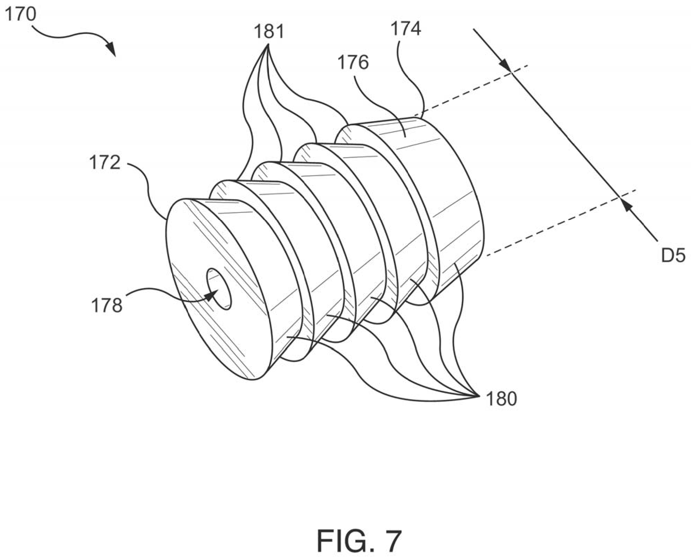

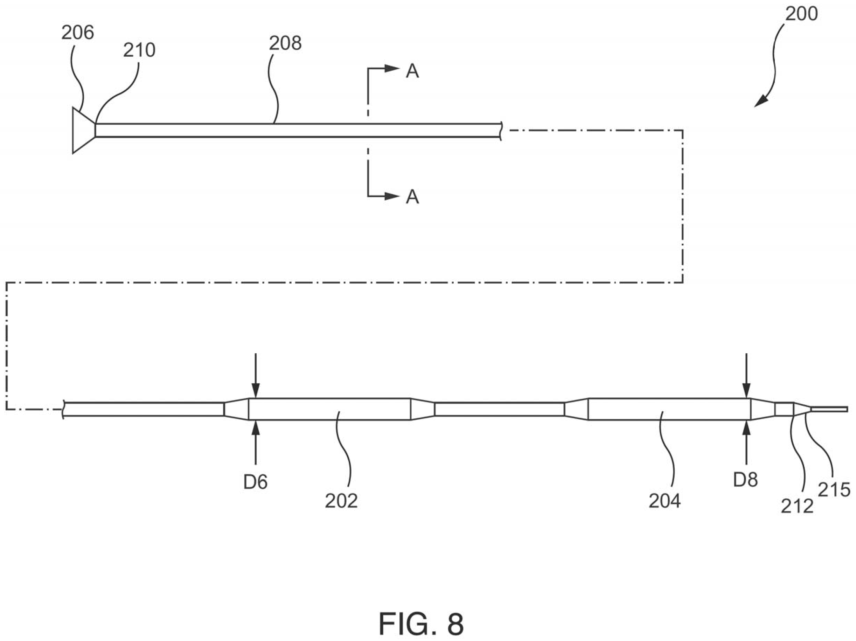

FIGS. 3-7 illustrate a repair device 100 according to an embodiment hereof. FIG. 3 illustrates the repair device 100 in a deployed configuration with a first anchor 102A in a closed state, a second anchor 102B in a closed state, and a tension member 106. FIG. 4 illustrates the repair device 100 in a delivery configuration. FIG. 5 illustrates the first anchor 102A or the second anchor 102B of the repair device 100 in the closed state. FIG. 6 illustrates the first anchor 102A or the second anchor 102B of the repair device 100 in an open state. FIG. 7 illustrates a lock of the repair device 100. FIGS. 8-11 illustrate an example delivery system for delivering and deploying the repair device 100.

In an embodiment, the repair device 100 includes the first anchor 102A, the second anchor 102B, the tension member 106, and a lock 170, as shown in FIG. 3. The first anchor 102A and the second anchor 102B are each made of a shape-memory material such that the first anchor 102A and the second anchor 102B may each transition between a closed state and an open state, as described below. The first anchor 102A and the second anchor 102B of the repair device 100 are each shape-set to return to the closed state. The repair device 100 may be delivered and deployed to a native valve by methods, procedures, and devices known to those familiar in the art.

FIG. 3 illustrates the repair device 100 in the deployed configuration with the first anchor 102A and the second anchor 102B each in the closed state and the tension member 106 in a deployed state. The repair device 100 in the deployed configuration has a second longitudinal distance LD2 between a second end 110 of the first anchor 102A and a first end 108 of the second anchor 102B. FIG. 4 illustrates the repair device 100 in the delivery configuration and disposed on a delivery catheter 200. The repair device 100 in the delivery configuration has a first longitudinal distance LD1 between the second end 110 of the first anchor 102A and the first end 108 of the second anchor 102B. In embodiments herein, the second longitudinal distance LD2 is shorter than the first longitudinal distance LD1.

In embodiments herein, the first anchor 102A and the second anchor 102B are each similar in structure and design. Therefore, the first anchor 102A and the second anchor 102B will be described together as the anchor 102. It is understood that the anchor 102 may be used for both the first anchor 102A and second anchor 102B. FIG. 5 shows an enlarged view of the anchor 102 in the closed state and FIG. 6 shows the anchor 102 in the open state. Each anchor 102 includes the first end 108, the second end 110, a body portion 120, and a claw portion 122. Each anchor 102 includes a first lumen 112 and a second lumen 114. The first lumen 112 extends from the first end 108 to the second end 110 and is generally defined by an inner surface 130 of the body portion 120. The second lumen 114 extends from the first end 108 to the second end 110 and is generally defined by an inner surface 132 of the claw portion 122 of the anchor 102. The body portion 120 includes a first longitudinal edge 126 and a second longitudinal edge 128 disposed generally opposite the first longitudinal edge 126 of the body portion 120. The second longitudinal edge 128 is defined by two longitudinal edges 128A, 128B that are adjacent to or abut each other in the closed state and are separated from each other in the open state, as explained below. The claw portion 122 includes a first longitudinal edge 134 and a second longitudinal edge 136 disposed generally opposite the first longitudinal edge 134 of the claw portion 122. The first longitudinal edge 134 of the claw portion 122 is generally coincident with the second longitudinal edge 128 of the body portion 120, and also includes two longitudinal edges 134A, 134B that are adjacent to or abut each other in the closed state and are separated in the open state. The claw portion 122 includes a plurality of fingers 146 separated longitudinally by a plurality of openings 148, as described below.

The first longitudinal edge 134 of the claw portion 122 is coupled to or coincident with the second longitudinal edge 128 of the body portion 120 such that the first lumen 112 of the body portion 120 and the second lumen of the claw portion 122 are generally parallel to each other. The first lumen 112 is configured to receive a balloon of a delivery catheter when the repair device 100 is in the delivery configuration. The second lumen 114 is configured to receive and grip a chordae of a native valve when the repair device is in the deployed configuration. The first lumen 112 has a first diameter D1 and the second lumen 114 has a second diameter D2 when the anchor 102 is in the closed state, as shown in FIG. 5. The first lumen 112 has a third diameter D3 and the second lumen 114 has a fourth diameter D4 when the anchor 102 is in the open state, as shown in FIG. 6. In embodiments herein, the first diameter D1 of the first lumen 112 in the closed state is smaller than the third diameter D3 of the first lumen 112 in the open state, and the second diameter D2 of the second lumen 114 in the closed state is smaller than the fourth diameter D4 of the second lumen 114 in the open state.

As shown in FIGS. 5-6, the body 120 of each anchor 102 is an arcuate shape. The body portion 120 is generally circular in shape in the closed state, as shown in FIG. 5. In greater detail, a first circumferential half 120A of the body 120, in cross section, extends in a first circumferential direction CD1 from the first longitudinal edge 126 to the second longitudinal edge 128A. A second circumferential half 120B of the body 120, in cross section, extends in a second circumferential direction CD2 from the first longitudinal edge 126 to the second longitudinal edge 128B. Described another way, the body 120 is generally circular in cross section *(i.e.,* is generally cylindrical) in the closed state, as shown in FIG. 5, and is generally C-shaped in the open state with the second longitudinal edges 128A, 128B circumferentially separate from each other, as shown in FIG. 6.

Similarly, and also shown in FIGS. 5-6, the claw portion 122 of each anchor 102 is an arcuate shape. The claw portion 122 is generally circular in shape in the closed state, as shown in FIG. 5. In greater detail, a first circumferential half 122A of the claw portion 122, in cross section, extends in the first circumferential direction CD1 from the first longitudinal edge 134A to the second longitudinal edge 136A. A second circumferential half 122B of the claw portion 122, in cross section, extends in the second circumferential direction CD2 from the first longitudinal edge 134B to the second longitudinal edge 136B. Described another way, the claw portion 122 is generally circular in cross section *(i.e.,* is generally cylindrical) in the closed state, as shown in FIG. 5. In the open state, as shown in FIG. 6, the first longitudinal edges 134A, 134B are separated from each other and the second longitudinal edges 136A, 136B are separated from each other. As such the claw portion, in the open state, is general oval in shape with portions of the oval missing where the first longitudinal edges 134A, 134B are separated from each other and the second longitudinal edges 136A, 136B are separated from each other.

The first circumferential half 120A of the body portion 120 further includes a first opening 140 adjacent the first end 108 and a second opening 142 adjacent the second end 110. The first opening 140 and the second opening 142 are configured to slidingly receive the tension member 106 such that the tension member 106 may be coupled to the anchor 102, as described below. The first opening 140 and the second opening 142 each extend from an outer surface 144 of the first circumferential half 120A to the lumen 112. In an embodiment, the first opening 140 and the second opening 142 may each have a generally oblong shape and may include a smoothed or beveled edge. In other embodiments, the first and second openings 140, 142 may be any shape suitable for the purposes described herein.

The claw portion 122 as noted above, the claw portion 122 includes a plurality of arcuate fingers 146 separated longitudinally by openings 148. In particular, the first circumferential half 122A includes a plurality of first fingers 146A extending in the first circumferential direction CD1 separated longitudinally by first openings 148A, and the second circumferential half 122B includes a plurality of second fingers 146B extending in the second circumferential direction CD2 separated longitudinally by second openings 148B.

While a specific number of fingers 146 and openings 148 of specific size and spacing are illustrated in FIGS. 3-7, this is not meant to be limiting, and a more or fewer fingers 146 and openings 148 of any suitable size and spacing may be utilized in any combination.

The tension member 106 includes a first end (not shown) and a second end 162, as shown in FIG. 3. In embodiments, the tension member 106 is configured to be selectively transitioned between a delivery state (FIG. 4) and the deployed state (FIG. 3). More specifically, in the embodiment of FIGS. 3-7, when the first anchor 102A and the second anchor 102B of the repair device 100 are each selectively coupled to, for example a chordae of a native heart valve, the tension member 106 may be transitioned from the delivery state to the deployed state by pulling or retracting the first end (not shown) of the tension member 106 in a first or proximal direction, indicated by an arrow P1, as shown in FIG. 3, to selectively move the second anchor 102B in the proximal direction towards the first anchor 102A, as described below. In embodiments, the tension member 106 extends from the first end (not shown), which is disposed outside the body of the patient, to the second end 162 which is coupled to the second opening 142 of the second anchor 102B, as shown in FIG. 3. In an embodiment, the tension member 106 may be woven between the first and second openings 140, 142 of the first and the second anchors 102A, 102B. In an embodiment, the second end 162 of the tension member 106 is woven over the first end 108 on the outer surface 144 of the first circumferential half 120A of the body portion 120 of the first anchor 102A of the repair device 100 and through the first opening 140 to the first lumen 112. The tension member 106 is then woven from the first lumen 112 through the second opening 142 to the outer surface 144 of the first circumferential half 120A of the body portion 120 of the first anchor 102A. In an embodiment, the tension member 106 may be selectively coupled to the first anchor 102A at the second opening 142 adjacent to the second end 110 of the first anchor 102A for example, with a sliding-knot or a slip-knot. The second end 162 of the tension member 106 is woven over the first end 108 and on the outer surface 144 of the first circumferential half 120A of the body portion 120 of the second anchor 102B. The tension member 106 is woven through the first opening 140 to the first lumen 112 of the second anchor 102B. The tension member 106 is then woven through the first lumen 112 to the second end 110 of the second anchor 102B and secured to the second anchor 102B. For example, the second end 162 of the tension member 106 may extend out of the second end 110 of the lumen 112, back over the outer surface 144 of the first circumferential half 120A of the body portion 120 of the second anchor 102B and tied in a knot to itself through the second opening 142. In other embodiments, the tension member 106 may be secured to the second anchor 102B by other methods suitable for the uses herein. For example, and not by way of limitation, the tension member 106 may be knotted around the second end 110 and second opening 142 of the second anchor 102B, a coupling device such as a crimp or other connector may secure the tension member 106 to the second anchor 102B, or other methods that would be apparent to those skilled in the art. In an embodiment, the tension member 106 may be a suture and may be formed of any suitable suture material, such as, but not limited to silk, nylon, polypropylene, polyester, polytetrafluoroethylene, or other materials suitable for the purposes described herein.

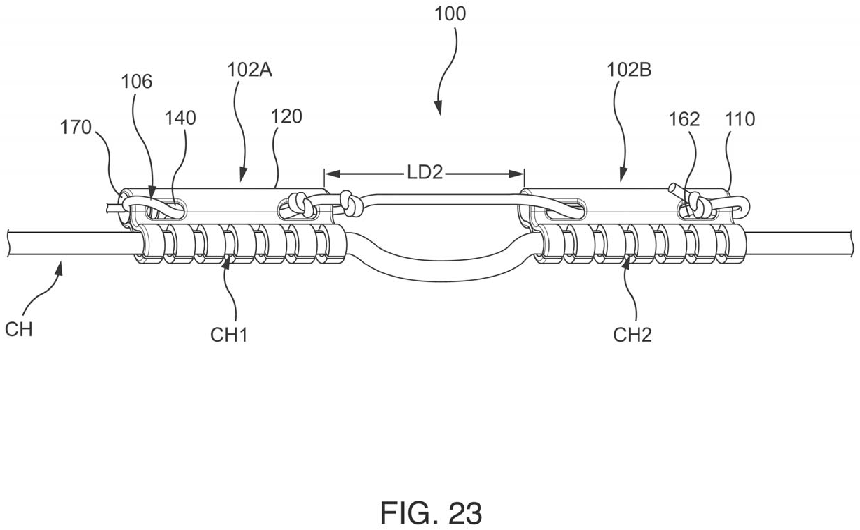

FIG. 7 illustrates the lock 170 according to an embodiment hereof. The lock 170 is configured to selectively secure the tension member 106 to the first anchor 102A to lock or retain the repair device 100 in the deployed configuration. In the embodiment of FIG. 7, the lock is generally tubular, including a first end 172, a second end 174, an outer surface 176, and a lumen 178 extending from the first end 172 to the second end 174. The lumen 178 is sized to slidingly receive the tension member 106 therethrough. The lock 170 is sized to be received within the first lumen 112 at the first end 108 of the first anchor 102A with the first anchor 102A in the closed state. The lock 170 has a fifth diameter D5 approximately equal to the first diameter D1 of the first lumen 112 when the first anchor 102A is in the closed state. With the first anchor 102A in the closed state, the lock 170 may be disposed within the first lumen 112 of the first anchor 102A such that the outer surface 176 of the lock 170 engages the inner surface 130 of the body portion 120 to frictionally couple the lock 170 therein. It will be understood that the lock 170 is a friction fit with the first lumen 112 of the first anchor 102A in the closed state. However, it shall be further understood that the lock 170 does impart a sufficient outward radial force onto the body portion 120 of the first anchor 102A to transition the first anchor 102A from the closed state. When the lock 170 is coupled to the first body portion 120 of the first anchor 102A, a portion of the tension member 106 is disposed between the outer surface 176 of the lock 170 and the inner surface 130 of the body portion 120 of the first anchor 102A such that the tension member 106 is frictionally secured to the first anchor 102A, as shown in FIGS. 3 and 23. In an embodiment, the outer surface 176 of the lock 170 may comprise a series of tapered segments 180 and shoulders 181, as shown in FIG. 7. The tapered segments 180 taper in the distal direction.

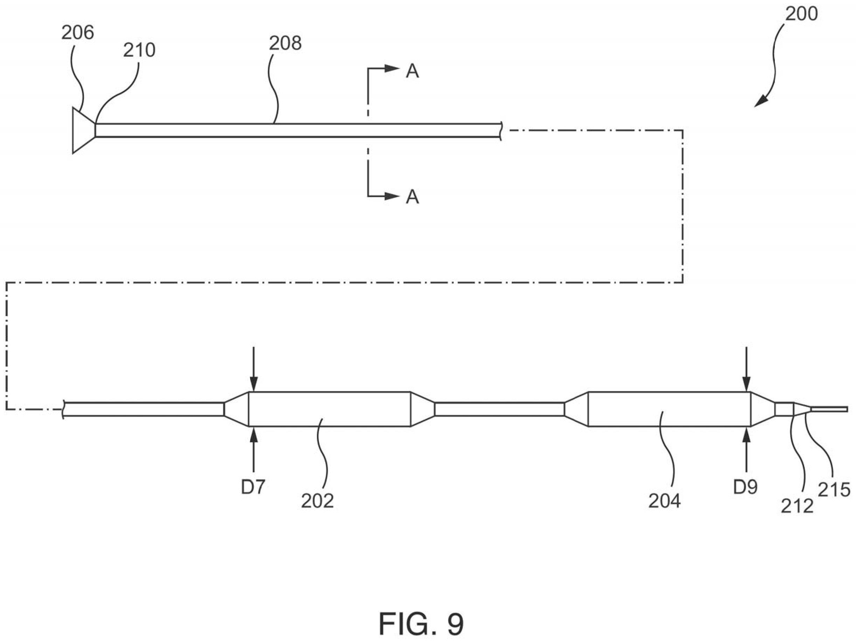

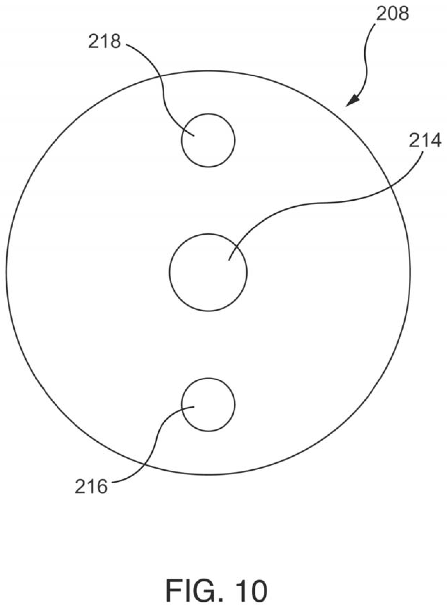

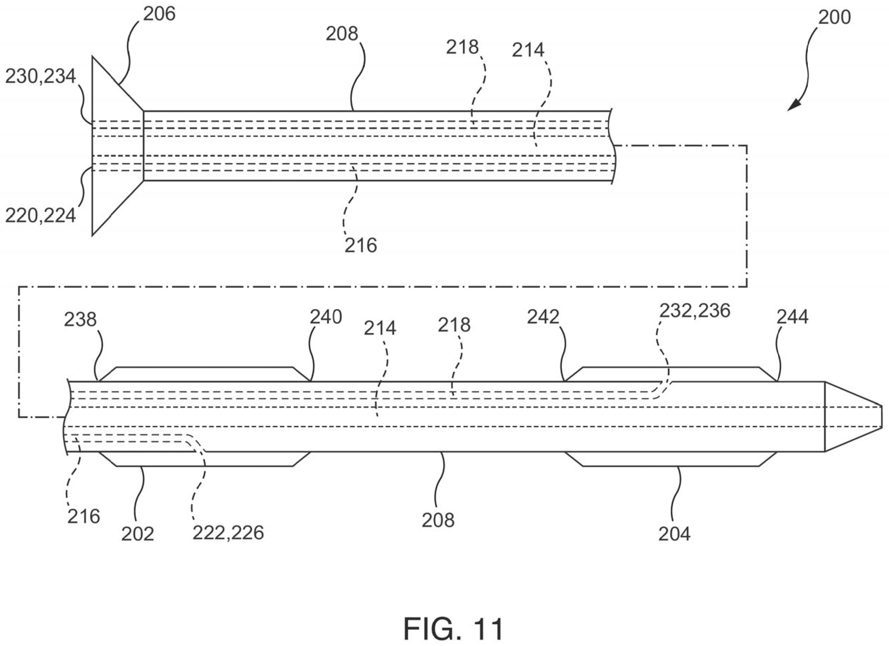

FIGS. 8-11 illustrate a delivery catheter 200 that may be utilized to deliver and deploy a heart valve repair device, such as the repair device 100, at the site of a chordae of a native heart valve. FIG. 8 shows a side view of the delivery catheter 200 with a first balloon 202 and a second balloon 204 each in an uninflated state. FIG. 9 shows a side view of the delivery catheter 200 with the first balloon 202 and the second balloon 204 each in an inflated state. FIG. 10 shows a cross section of the delivery catheter 200. FIG. 11 illustrates a longitudinal cross-sectional view of a distal portion of the delivery catheter 200. One skilled in the art will recognize that FIGS. 8-11 illustrate one example of a delivery catheter, that the systems, assemblies, and methods disclosed herein can be used with other delivery catheters, and that existing components illustrated in FIGS. 8-11 may be removed and/or additional components may be added to the delivery catheter 200. The delivery catheter 200 includes a hub 206, a shaft 208, the first balloon 202, and second balloon 204.

The shaft 208 may be any standard construction PTA catheter shaft, such as, but not limited to, multi-lumen or coaxial construction catheter shafts. The shaft 208 includes a proximal end 210 coupled to the hub 206 and a distal end 212. The distal end 212 is coupled to a distal tip 215. The shaft 208 may be made from any suitable material, such as, but not limited to polyamide (PA) and polyether block amide (PEBA).

A plurality of lumens are defined within the shaft 208, as shown in FIG. 10 and FIG. 11. The shaft 208 defines a guide wire lumen 214, a first inflation lumen 216, and a second inflation lumen 218. The guide wire lumen 214 extends from the proximal end 210 to the distal end 212 of the shaft 208. The guide wire lumen 214 is configured to slidably receive a guide wire therein.

The first inflation lumen 216 includes a proximal end 220 and a distal end 222. The proximal end 220 of the first inflation lumen 216 is in fluid communication with a proximal inflation port 224 disposed at the hub 206, as shown in FIG. 11. The distal end 220 of the first inflation lumen 216 is in fluid communication with a distal inflation port 226. The distal inflation port 226 is in fluid communication with an interior of the first balloon 202. The proximal end 220 of the first inflation lumen 216 enables fluid received through the proximal inflation port 224 to be delivered to the first balloon 202.

The second inflation lumen 218 includes a proximal end 230 and a distal end 232. The proximal end 230 of the second inflation lumen 218 is in fluid communication with a proximal inflation port 234 disposed at the hub 206. The distal end 232 of the second inflation lumen 218 is in fluid communication with a distal inflation port 236. The distal inflation port 236 is in fluid communication with an interior of the second balloon 204. The proximal end 230 of the second inflation lumen 218 enables fluid received through the proximal inflation port 234 to be delivered to the second balloon 204.

As would be understood by one skilled in the art, the hub 206 or other suitable fitting may be connected to a source of inflation fluid and may be of any construction or configuration without departing from the scope of the present invention.

The first balloon 202 includes a proximal end 238 coupled to the shaft 208 and a distal end 240 coupled to the shaft 208 distal of the proximal end 238. The first balloon 202 includes the uninflated state as shown in FIG. 8, and the inflated state shown in FIG. 9, wherein the first balloon 202 is inflated via inflation fluid under pressure. In embodiments, a diameter D6 of the first balloon 202 in the uninflated state is smaller than a diameter D7 of the first balloon 202 in the inflated state. Similarly, the second balloon 204 includes a proximal end 242 coupled to the shaft 208 and a distal end 244 and a distal end 244 coupled to the shaft 208 distal of the proximal end 242. The second balloon 204 is coupled to the shaft 208 distal of the first balloon 202. The second balloon 204 includes the uninflated state as shown in FIG. 8, and the inflated state shown in FIG. 9, wherein the second balloon 204 is inflated via inflation fluid. In embodiments, a diameter D8 of the second balloon 204 in the uninflated state is smaller than a diameter D9 of the second balloon 204 in the inflated state.

In embodiments herein, the diameters D6, D8 of the first and second balloons 202, 204, respectively, each in the uninflated state, is approximately equal to the first diameter D1 of the first lumen 112 of the anchor 102 of the repair device 100 with the first and second anchors 102A, 102B each in the closed state. In embodiments, the first balloon 202 and the second balloon 204 may each be a standard, a non-compliant, or a semi-compliant balloon constructed of any suitable materials such as, but not limited to polyethylene terephthalate (PET), nylon, polyether block amide (PEBA), or polyurethane. The first balloon 202 and the second balloon 204 may each be coupled to the shaft 208 by various methods including, but not limited to adhesives, fusing, bonding, or any other suitable method.

With an understanding of the components of the repair device 100 and the example delivery catheter 200 above, the interactions of the various components to repair a chordae of a native valve are described.

FIG. 4 shows the repair device 100 in the delivery configuration. The repair device 100 is disposed on a distal portion of the delivery catheter 200. The first balloon 202 of the delivery catheter 200 is in the uninflated state and is disposed within the body portion 120 of the first anchor 102A and is frictionally coupled to the first anchor 102A. The second balloon 204 of the delivery catheter 200 is in the uninflated state and is disposed within the body portion 120 of the second anchor 102B and is frictionally coupled to the second anchor 102B. In greater detail, an outer surface of the first balloon 202 engages the inner surface 130 of the body portion 120 of the first anchor 102A and an outer surface of the second balloon 204 of the delivery catheter 200 engages the inner surface 130 (FIG. 5) of the body portion 120 of the second anchor 102B to frictionally and releasably couple the delivery catheter 200 to the repair device 100.

The first balloon 202 and the second balloon 204 of the delivery catheter 200 may be inflated to transition the first anchor 102A and the second anchor 102B, respectively, of the repair device 100 from the closed state of FIG. 5 to the open state of FIG. 6. As shown in FIG. 11, inflation fluid is supplied under pressure to the proximal inflation ports 224, 234 of the first and second balloons 202, 204, respectively, at the hub 206. The inflation fluid travels from the proximal inflation port 224, through the first inflation lumen 216 and enters the first balloon 202 at the distal inflation port 226. The inflation fluid travels from the proximal inflation port 234, through the second inflation lumen 218 and enters the second balloon 204 at the distal inflation port 236. As the first balloon 202 and the second balloon 204 each receive inflation fluid under pressure, the first balloon 202 and the second balloon 204 each radially expands from the uninflated state to the inflated state. The first balloon 202 and the second balloon 204 each imparts an outward radial force on the corresponding inner surface 130 of the body portion 120 of the respective first and second anchors 102A, 102B to transition the first anchor 102A and the second anchor 102B each to the open state. When the first and second anchors 102A, 102B are in the open state, as shown in FIG. 6, the lumen 114 of the claw portion 122 is accessible via the opening between the tips of the fingers 146A and 146B *(i.e.,* between the separated second longitudinal edges 136A, 136B of the claw portion 122) such that the second lumen 114 may receive tissue, for example chordae of a native heart valve, therein.

After receiving tissue in the second lumen 114, the first and second balloons 202, 204 of the delivery catheter 200 may be uninflated to transition the first and second anchors 102A, 102B from the open state to the closed state. Referring again to FIG. 11, supply pressure on the inflation fluid at the proximal inflation ports 224, 234 of the first and second balloons 202, 204, respectively, is released. The inflation fluid travels from the first balloon 202 via the distal inflation port 226 through the first inflation lumen 216 and exits the delivery catheter 200 via the proximal inflation port 224 at the hub 206. For the second balloon 204, the inflation fluid travels via the distal inflation port 236 through the first inflation lumen 218 and exits the delivery catheter 200 via the proximal inflation port 234 at the hub 206. When inflation fluid exits the first and second balloons 202, 204 each balloon 202, 204 radially contracts from the inflated state to the uninflated state. Accordingly, the first anchor 102A and the second anchor 102B of the repair device 100 each transitions from the open state to the closed state, thereby capturing the tissue (chordae) within the second lumen 114.

With the repair device 100 coupled to the chordae, as described above, and the tension member 106 in the delivery state, the distance between the second end 110 of the first anchor 102A and the first end 108 of the second anchor 102B is the second longitudinal distance LD2, as shown in FIG. 4. The tension member 106 may then be pulled to transition the repair device 100 to the deployed state. In particular, pulling the tension member 106 in the proximal direction P1 pulls the second anchor 102B in the proximal direction P1. Thus, the second anchor 102B is proximally retracted relative to the first anchor 102A, thereby reducing the longitudinal distance between the first and second anchors 102A, 102B from the first longitudinal distance LD1 to the second distance LD2. With the chordae captured in the first and second anchors 102A, 102B, the shortening of the distance between the anchors 102A, 102B also shortens or tightens the chordae, as shown in more detail below. The shortened chordae bring the native valve leaflets of the native valve closer together to reduce or eliminate valve regurgitation.

The delivery catheter 200 may be removed prior to or after shortening the distance between the anchors 102A, 102B. With the delivery catheter 200 removed, and tension maintained on the tension member 106, the lock 170 may be advanced and disposed within the first lumen 112 of the first anchor 102A to couple the lock 170 to the first anchor 102A and to secure the tension member 106 to the first anchor 102A. More particularly, the lock 170 is advanced over the tension member 106 and into the first lumen 112 of the first anchor 102A, thereby trapping the tension member 106 is between the outer surface 176 of the lock 170 and the inner surface 130 of the body portion 120 of the first anchor 102A to secure a portion of the tension member 106 to the first anchor 102A. Securing the lock 170 within the first lumen 112 with the tension member 106 trapped between the outer surface 176 of the lock and the inner surface 130 of the first anchor 102A locks the repair device 100 in the deployed configuration with the second longitudinal distance LD2 between the second end 110 of the first anchor 102A and the first end 108 of the second anchor 102B.

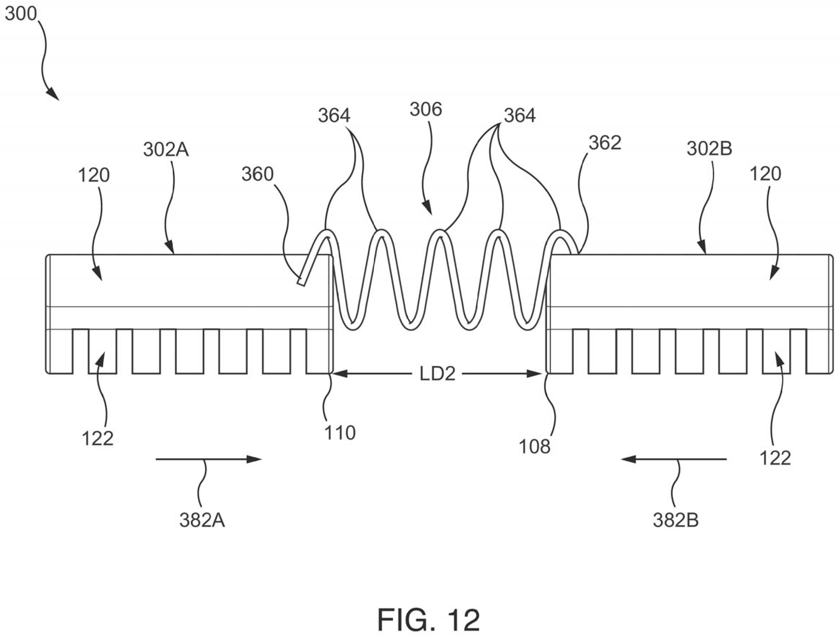

FIGS. 12-13 illustrate a repair device 300 according to an embodiment hereof. The repair device 300 is similar to the repair device 100 shown in FIGS. 3-11 except as described herein. Therefore, all of the details described above are incorporated into the description of FIGS. 12-13 and the same reference numerals are used. Thus, generally, as described above, the repair device 300 includes a first anchor 302A and a second anchor 302B. However, the repair device 300 differs from the repair device 100 in that the repair device 300 includes a helical coil spring tension member 306. Further, the body portions 120 of the first anchor 302A and the second anchor 302B do not need to include the first and second openings 140, 142, although they could include them. Still further, the repair device 300 does not include the lock 170.

Thus, the first anchor 302A and the second anchor 102B are the same as the first anchor 102A and the second anchor 102B, respectively, except that the body portions 120 do not include the first and second openings 140, 142. Therefore, a detailed description of the first anchor 302A and the second anchor 302B will not be repeated here. In embodiments herein, the first anchor 302A and the second anchor 302B of the repair device 300 each includes the body portion 120, the claw portion 122, the first lumen 112, and the second lumen 114, as described above.

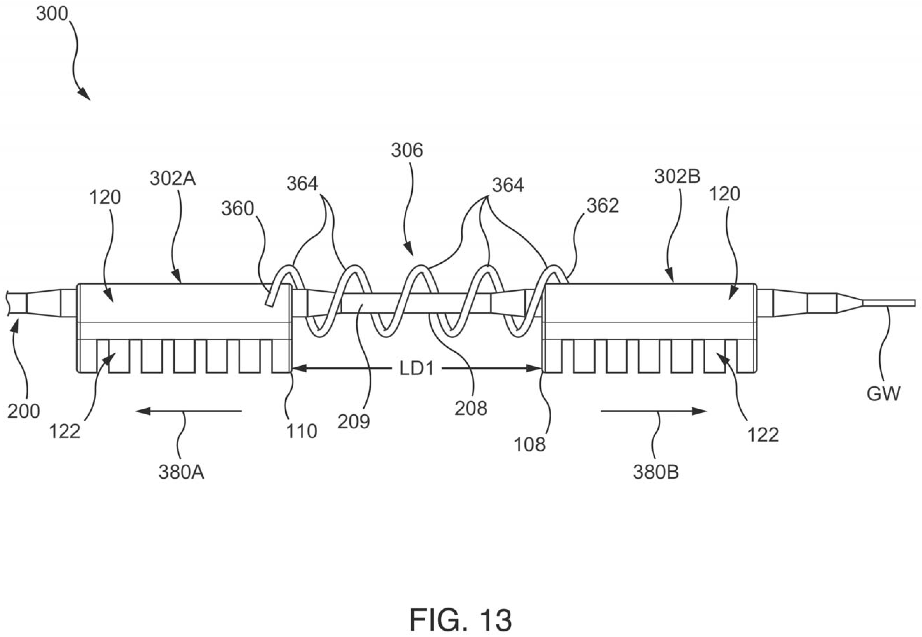

In embodiments, the tension member 306 is a tension spring including a first end 360, a second end 362 and a plurality of helical coils 364. The first end 360 of the tension member 306 is coupled to the first body portion 120 of the first anchor 102A and the second end 362 of the tension member 306 is coupled to the first body portion 120 of the second anchor 102B. The tension member 306 is configured to maintain the second longitudinal distance LD2 between the second end 110 of the first anchor 302A and the first end 108 of the second anchor 302B when the tension member 306 is in the deployed state and the repair device 300 is in the deployed configuration, as shown in FIG. 12. FIG. 13 illustrates the repair device 300 in the delivery configuration and the tension member 306 is in the delivery state. The second end 110 of the first anchor 302A and the first end 108 of the second anchor 302B are spaced a first longitudinal distance LD1 when the repair device 300 is in the delivery configuration. The second longitudinal distance LD2 is smaller than the first longitudinal distance LD1. In embodiments of FIGS. 12-13, the tension member 306 is placed in tension for delivery, with the first anchor 102A and the second anchor 102B each moved away from each other, as indicated by arrows 380A, 380B in FIG. 13. When the tension member 306 transitions to the deployed state, the tension member 306 shortens or contracts, thereby pulling the first anchor 302A and the second anchor 302B toward each other as indicated by the arrows 382A, 382B, and returning the distance between the first and second anchors 302A, 302B to the second longitudinal distance LD2, as shown in FIG. 12. The amount of force with which the tension member 306 attempts to draw or pull the first anchor 302A and the second anchor 302B towards each other may be selected by the spring design characteristics of the tension member 306 and may therefore be customized for various anatomies.

The tension member 306 may be formed of various materials including, but not limited to nickel-titanium alloys (e.g., NITINOL), or other suitable materials. The tension member 306 may be coupled to the first anchor 302A and the second anchor 302B by methods such as, but not limited to welding, fusing, adhesives, riveting, and interference fit. While the tension member 306 is illustrated in FIG. 12 with a specific free length, coil diameter, wire diameter, coil pitch, and coil spacing, this is not meant to be limiting, and the tension member 306 may include other free lengths, coil diameters, wire diameter, pitches, and coil spacings.

The tension member 306 may be retained in the delivery state, shown in FIG. 13, by various methods, non-limiting examples including an outer sheath disposed over the tension member 306 and configured to frictionally retain the tension member 306 in the delivery state. In another embodiment, a portion 209 of the catheter shaft 208 of the catheter 200 disposed between the first balloon 202 and the second balloon 204 and a guidewire GW disposed therein may include sufficient longitudinal stiffness or rigidity to maintain the tension member 306 in the delivery state. Stated another way, when the repair device 300 is in the delivery configuration, the first balloon 202 in the uninflated state is disposed within and frictionally coupled to the first anchor 302A, the second balloon 204 in the uninflated state is disposed within and frictionally coupled to the second anchor 302B, and the longitudinal stiffness of the portion 209 of the catheter shaft 208 disposed between the first balloon 202 and the second balloon 204 and the guidewire GW disposed therein combine to maintain the first distance LD1 between the first anchor 302A and the second anchor 302B during tracking through the vasculature and positioning of the first and second anchors 302A, 302B at the desired treatment location.

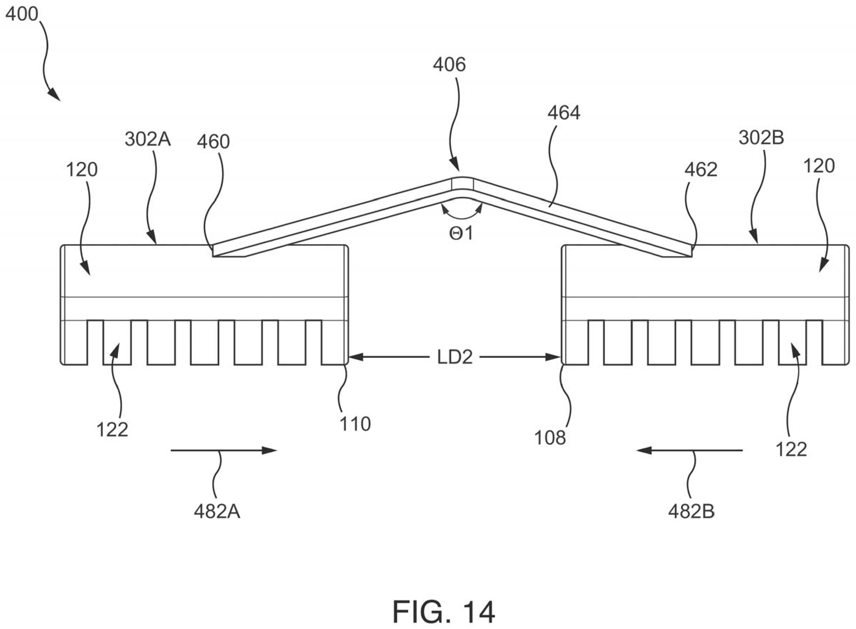

FIGS. 14-15 illustrate a repair device 400, according to an embodiment hereof. The repair device 400 is similar to the repair device 300 shown in FIGS. 12-13 except as described herein. Therefore, all of the details described above are incorporated into the description of FIGS. 12-13 and the same reference numerals are used. Thus, generally, as described above, the repair device 400 includes the first anchor 302A and the second anchor 302B. However, the repair device 400 differs from the repair device 300 of FIGS. 12-13 in that repair device 400 includes a shape memory tension member 406.

In embodiments, the tension member 406 includes a first end 460, a second end 462 and a V-shaped connector 464. The first end 460 of the tension member 406 is coupled to the body portion 120 of the first anchor 302A. The second end 462 of the tension member 406 is coupled to the body portion 120 of the second anchor 302B. The tension member 406 is shape-set to the such that the distance between the second end 110 of the first anchor 302A and the first end 108 of the second anchor 302B is the second longitudinal distance LD2. Thus, the tension member 406 is shape-set to the deployed state. In the embodiment of a V-shaped connector 464, the V-shaped connector 464 is shape-set to an angle Θ1 that is smaller than an angle Θ2 of the V-shaped connector 464 in the delivery state. In other words, the first angle Θ1 of the V-shaped connector 464 in the deployed state is more acute than the second angle Θ2 of the V-shaped connector 464 is the delivery state.

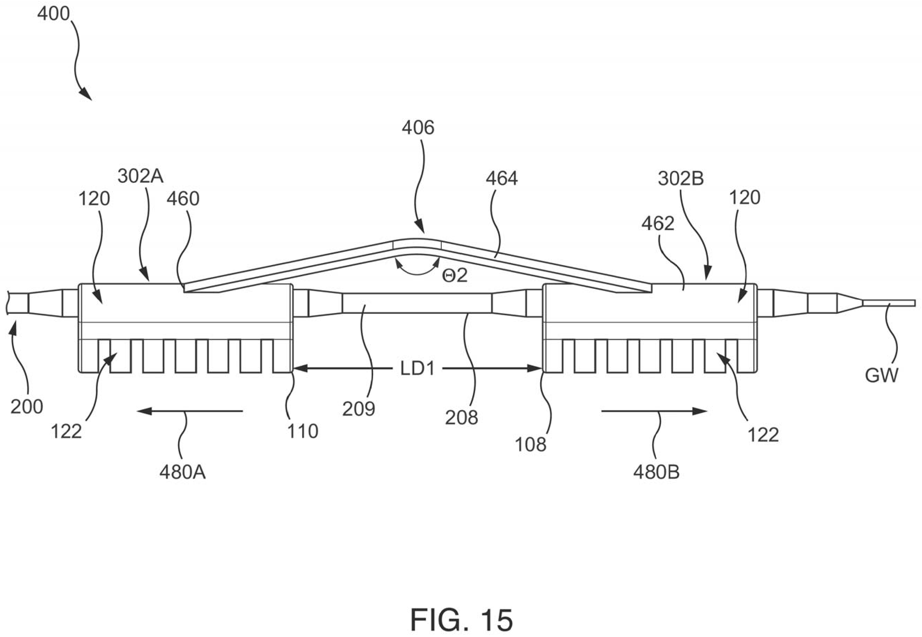

Thus, the tension member 406 is placed in tension for delivery such that the first anchor 302A and the second anchor 302B are moved away from each other, as indicated by arrows 480A, 480B in FIG. 15. When the tension member 406 transitions to the deployed state, the tension member 406 shortens or contracts longitudinally by folding *(i.e.,* decreasing from the second angle Θ2 to the first angle Θ1), thereby pulling the first anchor 302A and the second anchor 302B toward each other as indicated by the arrows 482A, 482B, and returning the distance between the first and second anchors 302A, 302B to the second longitudinal distance LD2, as shown in FIG. 14. The amount of force with which the tension member 406 attempts to draw or pull the first anchor 302A and the second anchor 302B towards each other may be selected by design characteristics of the tension member 406.

The tension member 406 may be formed of various shape memory materials including, but not limited to, a nickel titanium alloy or nitinol, various polymers, or a so-called super alloy, which may have a base metal of nickel, cobalt, chromium, or other metal. While the tension member 406 is illustrated in FIGS. 14-15 with a specific free length and shape, this is not meant to be limiting, and the tension member 406 may include other free lengths and shapes suitable for the purposes described herein and customizable to conform to native anatomies.

The tension member 406 may be retained in the delivery state for delivery by various methods as described above, non-limiting examples including an outer sheath disposed over the tension member 406 and configured to retain the tension member 406 in the delivery state, or when the repair device 400 is in the delivery configuration, the first balloon 202 in the uninflated state is disposed within and frictionally coupled to the first anchor 302A, the second balloon 204 in the uninflated state is disposed within and frictionally coupled to the second anchor 302B, and the longitudinal stiffness of the portion 209 of the catheter shaft 208 disposed between the first balloon 202 and the second balloon 204 combine to maintain the first distance LD1 between the first anchor 302A and the second anchor 302B during tracking through the vasculature and positioning of the first and second anchors 302A, 302B at the desired treatment location..

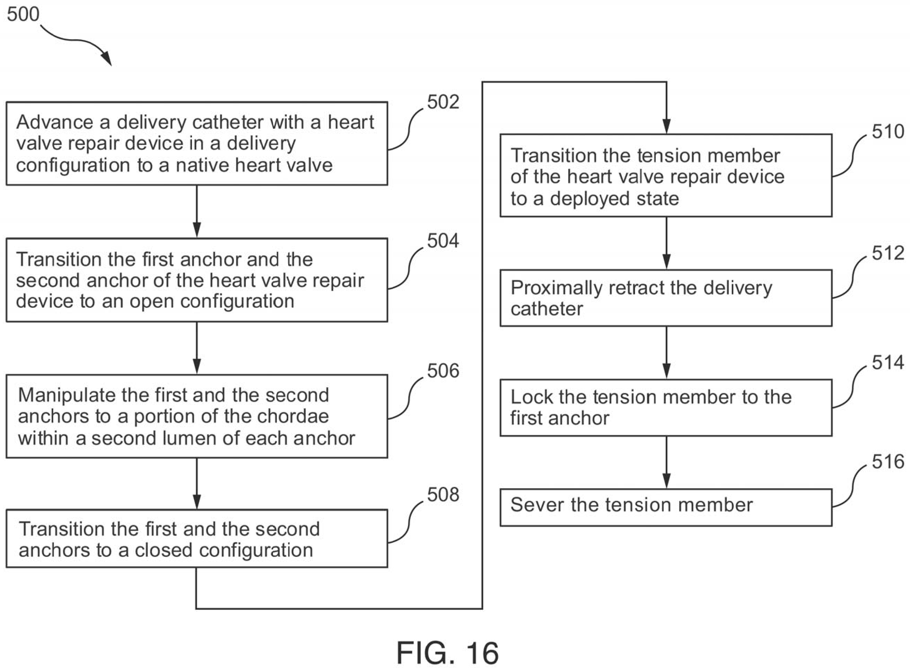

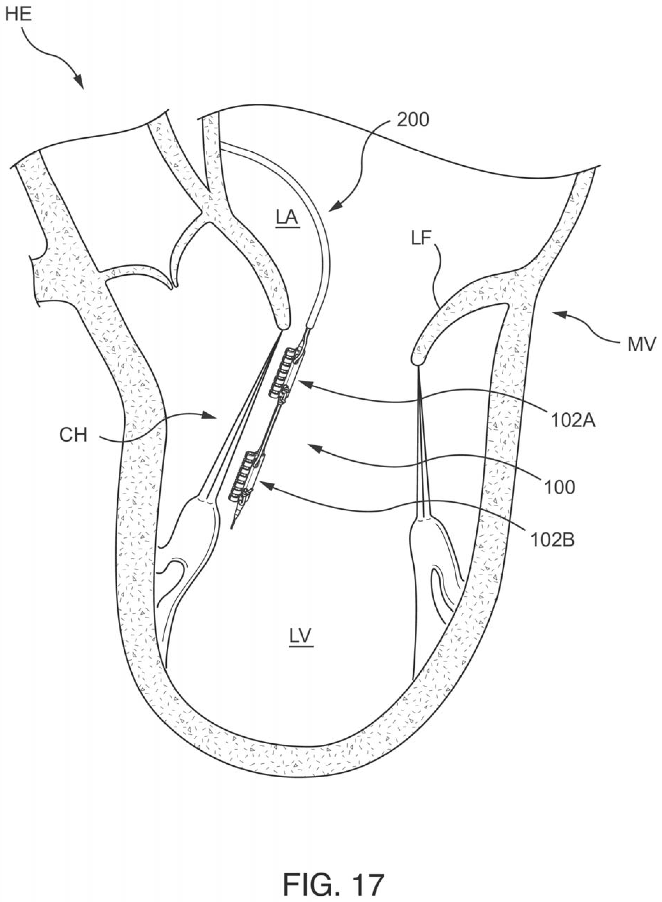

FIG. 16 is a flow chart showing a method 500 for repairing a chordae of a native mitral valve with the repair device 100. FIGS. 17-23 shows steps of the method 500 using the repair device 100 *in situ.* While described herein as deploying the repair device 100 at the site of a native mitral valve, this is not meant to be limiting, and the method described herein may be utilized at other native heart valve locations. Further, although the method 500 is described with respect to the repair device 100, the method 500 may be used with the repair device 300 or the repair device 400. One skilled in the art will realize that FIGS. 16-23 illustrate one example of a method of using the repair device and that certain steps of the method may be removed or combined, and that additional steps of the method may be performed in keeping with scope of the present disclosure.

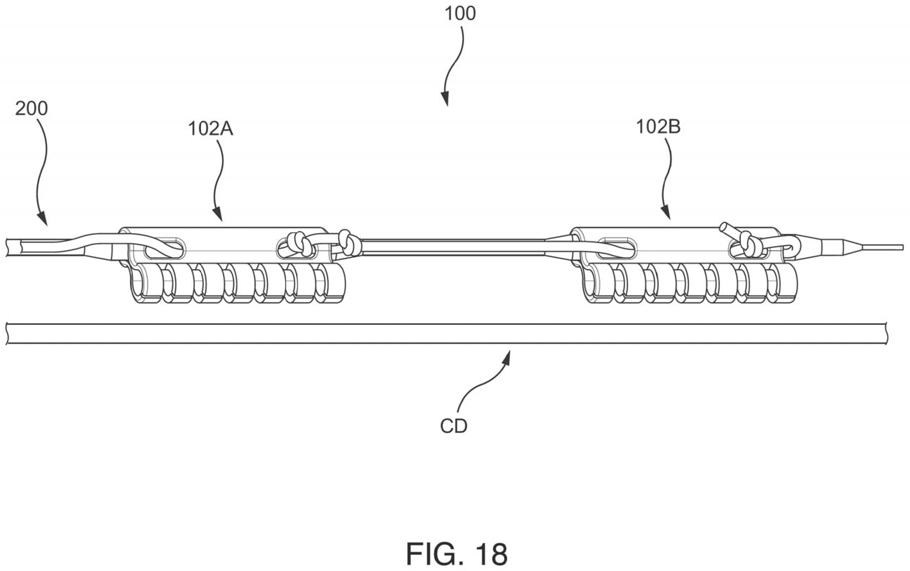

In a step 502 of the method 500, shown in FIG. 17, the repair device 100 is advanced through the vasculature of a patient to a native mitral valve MV of a heart HE utilizing established methods and procedures. The first anchor 102A and the second anchor 102B, each in the closed state, are disposed within a left ventricle LV, adjacent to a chordae CH of a native mitral valve MV, as shown in FIG. 17 and in an enlarged view in FIG. 18. In the embodiment of FIGS. 17-23, the delivery catheter 200 with the repair device 100 disposed thereon is delivered in a transeptal approach through the transeptal wall, into the left atrium LA, through the native mitral valve MV, and into the left ventricle LV. Thus, in the embodiment shown, the first anchor 102A is disposed closer to the native mitral valve MV than the second anchor 102B. While the procedure is described herein as gaining access to the chordae via the left atrium and through the native mitral valve, if a different approach is utilized, for example a transapical approach, it shall be understood that the second anchor 102B may be disposed closer to the native mitral valve MV than the first anchor 102A.

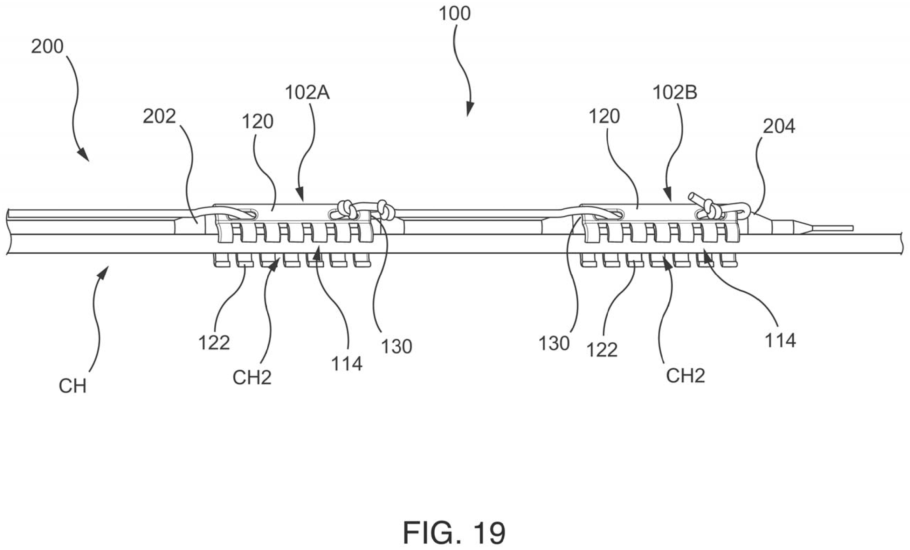

In a step 504 of the method 500, as shown in FIG. 19, the first balloon 202 and the second balloon 204 of the delivery catheter 200 are each inflated to transition the first anchor 102A and the second anchor 102B of the repair device 100 each to the open state. In greater detail, inflation fluid is supplied under pressure to the first ballon 202 and the second balloon 204. As the first balloon 202 and the second balloon 204 each transitions to the inflated state, the first balloon 202 and the second balloon 204 each imparts an outward radial force on the corresponding inner surface 130 of the body portion 120 of the first and second anchors 102A, 102B, respectively, to transition the first anchor 102A and the second anchor 102B to the open state.

In a step 506 of the method 500, the catheter 200 is manipulated such that a first portion CH1 of the chordae CH of the native mitral valve MV is received within the second lumen 114 of claw portion 122 of the first anchor 102A and a second portion CH2 of the chordae CH is received within the second lumen 114 of the claw portion 122 of the second anchor 102B, as shown in FIG. 19.

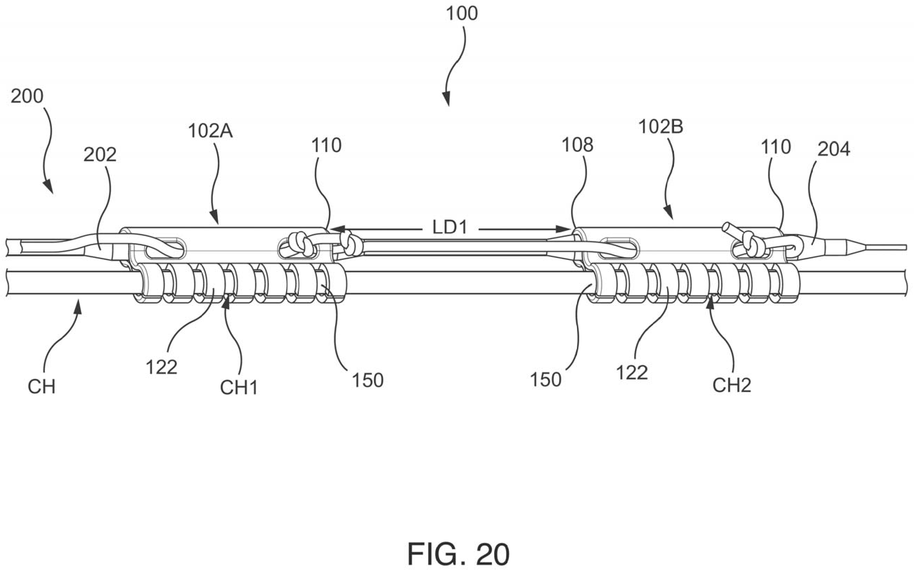

In a step 508 of the method 500, the fluid pressure on the first balloon 202 and the second balloon 204 is released such that the first balloon 202 and the second balloon 204 each transitions from the inflated state to the uninflated state, and the first anchor 102A and the second anchor 102B each transitions from the open state to the closed state. As shown in FIG. 20, when the first anchor 102A and the second anchor 102B are each in the closed state with a corresponding portions CH1, CH2 of the chordae CH disposed therein. The inner surface 150 of the claw portion 122 of the first anchor 102A and the second anchor 102B each engages the respective portion CH1, CH2 of an outer surface of the chordae CH to frictionally couple the first anchor 102A to the first portion CH1 of the chordae CH and the second anchor 102B to the second portion CH2 of the chordae CH. At this stage of the method, the first end 108 of the second anchor 102B is disposed the first longitudinal distance LD1 from the second end 110 of the first anchor 102A. It shall be understood that the first and second anchors 102A, 102B may be repositioned for optimal placement prior transitioning the repair device 100 to the deployed state.

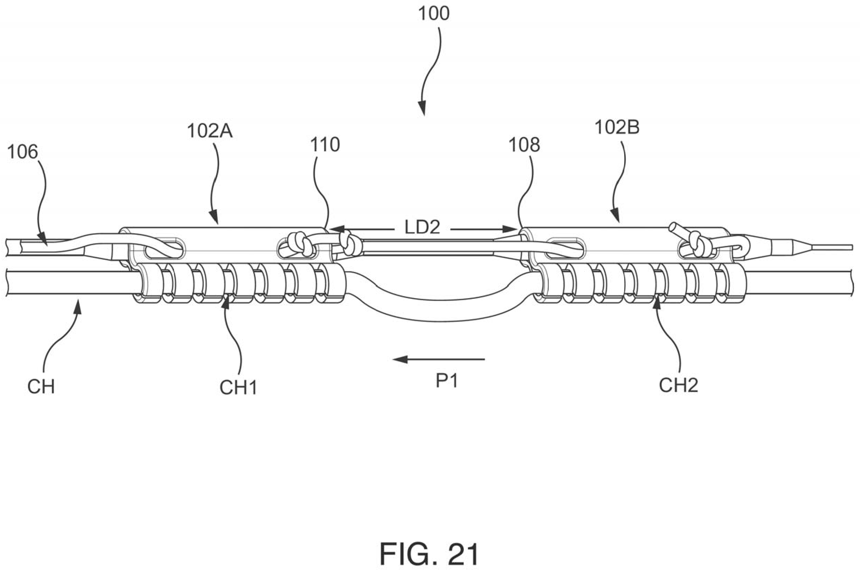

In a step 510 of the method 500, with the repair device 100 coupled to the chordae CH, the tension member 106 of the repair device 100 is transitioned to the deployed state. In embodiments, the proximal end (not shown) of the tension member 106 is proximally retracted, as indicated by an arrow P1 in FIG. 21. Tension in the proximal direction P1 on the tension member 106 pulls the second anchor 102B in the proximal direction P1. Stated another way, tension on the tension member 106 moves the second anchor 102B towards the first anchor 102A. Accordingly, the second portion CH2 of the chordae CH is moved in the direction P1 towards the first portion CH1 of the chordae CH, thereby shortening or tightening the chordae CH. With the tension member 106 under tension, the distance between the second end 110 of the first anchor 102A and the first end 108 of the second anchor 102B is the second longitudinal distance LD2, where the second longitudinal distance LD2 is smaller than the first longitudinal distance LD1.

In a step 512 of the method 500, with tension on the tension member 106, the delivery catheter 200 is removed.

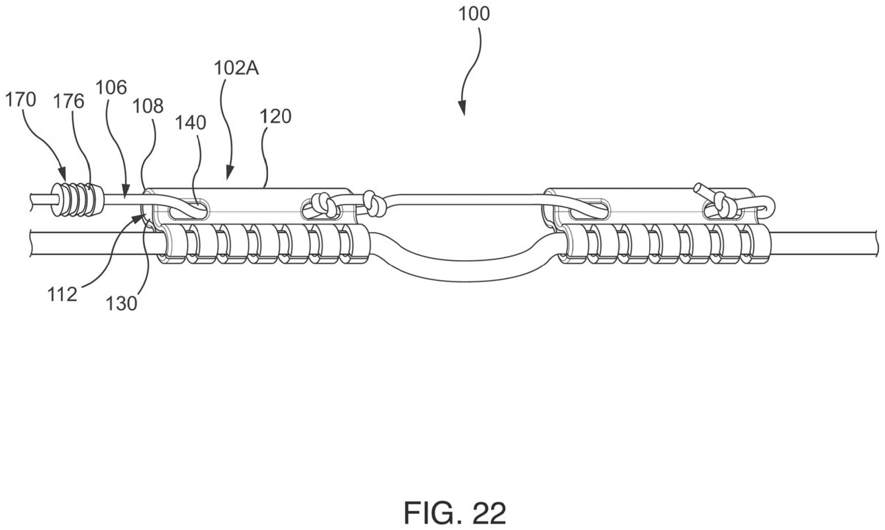

In a step 514 of the method 500, as shown in FIG. 22, the lock 170 of the repair device 100 is advanced over the tension member 106 and disposed within the first lumen 112 at the first end 108 of the first anchor 102A to couple the lock 170 to the first anchor 102A and to secure the tension member 106 to the first anchor 102A. In an embodiment a pusher (not shown) with an opening sized to the receive the tension member 106 includes a distal surface to push the lock 170 over the tension member 106 and into the first anchor 102A. When the lock 170 is pushed into the first anchor 102A, a portion of the tension member 106 is disposed between the outer surface 176 of the lock 170 and the inner surface 130 of the body portion 120 to secure a portion of the tension member 106 to the first anchor 102A, as shown in FIG. 23. With the tension member 106 locked in tension by the locking member 106, the second anchor 102B is retained at the second longitudinal distance LD2 relative to the first anchor 102A and the chordae CH remains in a shortened or tightened state. In other embodiments, instead of using a lock, the slide-knot or slip-knot coupling the tension member 106 to the first anchor 102A enables the tension member 106 to be pulled in the proximal direction P1, but prevents the tension member 106 from moving in the distal direction opposite the proximal direction. Therefore, once the tension member 106 is pulled in the proximal direction P1 such that the first anchor 102A first direction manner in which the tension member 106 is coupled to the first anchor 102A and the second anchor 102B are spaced the second longitudinal distance LD2 from each other, the slide-knot or slip-knot prevents the second anchor 102B from moving in the distal direction, thereby retaining the second anchor 102B at the second longitudinal distance LD2 relative to the first anchor 102A and the chordae CH in the shortened or tightened state

In a step 516 the tension member 106 is severed adjacent to the first end 108 of the first anchor 102A using established procedures.

With the repair device 100 deployed at the site of a chordae CH of a native mitral valve MV (FIG. 13), the first anchor 102A is coupled to the first portion CH1 of the chordae CH, the second anchor 102B is coupled to the second portion CH2 of the chordae, the second end 162 of the tension member is coupled to the second end 110 of the second anchor 102B, and a proximal portion of the tension member 106 is coupled to the first anchor 102A. The tension member 106 is maintained in tension such that the second anchor 102B is retained at the second longitudinal distance LD2 and the chordae CH is retained in the shortened or tightened state as shown in FIG. 23.

Although the method 500 was described with respect to repair device 100, the method applies equally to the repair device 300 and the repair device 400. However, due to the different designs thereof, the steps of tightening the tension member, advancing the lock, and severing the tension member are not needed. Instead, after closing the first and second anchors 302A, 302B onto the chordae, the catheter 200 is withdrawn such that the tension member 306 or the tension member 406 reverts to the deployed state with the first and second anchors 302A, 302B spaced the second distance LD2 from each other, thereby shortening or tightening the chordae. As previously described, the tension members for embodiments of repair devices 300 and 400 may be retained in the delivery state for delivery by various methods, non-limiting examples including an outer sheath disposed over the tension member and configured to retain the tension member in the delivery state, or when the repair device 300, 400 is in the delivery configuration, the first balloon 202 in the uninflated state is disposed within and frictionally coupled to the first anchor 302A, the second balloon 204 in the uninflated state is disposed within and frictionally coupled to the second anchor 302B, and the longitudinal stiffness of the portion 209 of the catheter shaft 208 disposed between the first balloon 202 and the second balloon 204 combine to maintain the first distance LD1 between the first anchor 302A and the second anchor 302B.

It should be understood that, depending on the example, certain acts or events of any of the processes or methods described herein may be performed in a different sequence, may be added, merged, or left out altogether (*e.g.,* all described acts or events may not be necessary to carry out the techniques). For example, in embodiments of repair devices wherein the repair device is shape-set to return to the deployed state, methods of repairing a chordae of a native mitral valve with, for example the repair device 300 or the repair device 400 may omit the step 514 and the step 516.

While various embodiments have been described above, it should be understood that they have been presented only as illustrations and examples of the present technology, and not by way of limitation. It will be apparent to persons skilled in the relevant art that various changes in form and detail may be made therein without departing from the spirit and scope of the present technology. Thus, the breadth and scope of the present technology should not be limited by any of the above-described embodiments but should be defined only in accordance with the appended claims and their equivalents. It will also be understood that each feature of each embodiment discussed herein, and of each reference cited herein, may be used in combination with the features of any other embodiment. All patents and publications discussed herein are incorporated by reference herein in their entirety.

The following examples are illustrative of the techniques described herein.

Example 1. A heart valve repair device having a delivery configuration and a deployed configuration, the heart valve repair device comprising: a first anchor having an open state and a closed state; a second anchor having an open state and a closed state; and a tension member coupled to the first anchor and the second anchor; wherein with the heart valve repair device in the delivery configuration, the first anchor is spaced a first longitudinal distance from the second anchor; and wherein the tension member is configured to translate the second anchor relative to the first anchor such that the first anchor is spaced a second longitudinal distance from the second anchor, the second longitudinal distance being shorter than the first longitudinal distance.

Example 2. The heart valve repair device of Example 1, wherein the first anchor includes a first body portion and a first claw portion, wherein the first anchor in the open state is configured to receive a first portion of a chordae therein, and wherein the first anchor in the closed state is configured to grasp the first portion of the chordae.

Example 3. The heart valve repair device of Example 2, wherein the second anchor includes a second body portion and a second claw portion, wherein the second anchor in the open state is configured to receive a second portion of the chordae therein, and wherein the second anchor in the closed state is configured to grasp the second portion of the chordae.

Example 4. The heart valve repair device of Example 3, wherein the tension member is configured to translate the second anchor relative to the first anchor with the chordae grasped by the first anchor and the second anchor such that the chordae is shortened.

Example 5. The heart valve repair device of Example 1, wherein the first anchor and the second anchor are shape-set to the closed state.

Example 6. The heart valve repair device of Example 1, wherein the tension member comprises a suture slidably coupled to the first anchor and fixedly secured to the second anchor such that pulling the tension member pulls the second anchor towards the first anchor.

Example 7. The heart valve repair device of Example 1, further comprising a lock configured to secure the tension member to the first anchor with the heart valve repair device in the deployed configuration such that the first anchor is spaced the second longitudinal distance from the second anchor.

Example 8. The heart valve repair device of Example 1, wherein the tension member includes a first end fixedly secured to the first anchor and a second end fixedly secured to the second anchor, wherein the tension member is shape-set such that the first anchor is spaced the second longitudinal distance from the second anchor, and wherein in the delivery configuration, the tension member is placed in tension such that the first anchor is spaced the first longitudinal distance from the second anchor.

Example 9. The heart valve repair device of Example 8, wherein the tension member is a spring.

Example 10. The heart valve repair device of Example 8, wherein the tension member is a shape-set V-shaped connector.

Example 11. A method of repairing a native heart valve comprising: delivering a heart valve repair device to a site of the native heart valve, wherein the heart valve repair device comprises a first anchor, a second anchor, and a tension member, wherein the heart valve repair device is delivered to the site in a delivery configuration; grasping a first portion of a chordae with the first anchor; grasping a second portion of the chordae with the second anchor, wherein the first anchor is spaced a first longitudinal distance from the second anchor; and with the first portion and the second portion of the chordae grasped by the first anchor and the second anchor, respectively, transitioning the tension member to a deployed state such that the first anchor is spaced a second longitudinal distance from the second anchor, wherein the second longitudinal distance is shorter than the first longitudinal distance, thereby shortening or tightening the chordae.

Example 12. The method of Example 11, wherein grasping the first portion of the chordae with the first anchor comprises: delivering the first anchor to a position adjacent the first portion of the chordae in a closed state of the first anchor; transitioning the first anchor to an open state; receiving the first portion of the chordae in the first anchor with the first anchor in the open state; and transitioning the first anchor to a closed state to grasp the first portion of the chordae.

Example 13. The method of Example 11, wherein grasping the second portion of the chordae with the second anchor comprises: delivering the second anchor to a position adjacent the second portion of the chordae in a closed state of the second anchor; transitioning the second anchor to an open state; receiving the second portion of the chordae in the second anchor with the second anchor in the open state; and transitioning the second anchor to a closed state to grasp the second portion of the chordae.

Example 14. The method of Example 11, wherein the tension member is a suture slidably coupled to the first anchor and fixed coupled to the second anchor, wherein transitioning the tension member to the deployed state comprises pulling the tension member such that the second anchor is pulled towards the first anchor.

Example 15. The method of Example 11, further comprising deploying a lock to lock the tension member in the deployed state.

Example 16. The method of Example 11, further comprising, after transitioning the tension member to the deployed state, severing the tension member.

Example 17. The method of Example 11, wherein the tension member includes a first end fixedly secured to the first anchor and a second end fixedly secured to the second anchor, wherein in the delivery configuration the tension member is tensioned such that the first anchor is spaced the first longitudinal distance from the second anchor, and wherein the tension member is shape-set to the deployed state such that transitioning the tension member to the deployed state comprises releasing the tension member such that the tension member returns to the deployed state.

Example 18. The method of Example 17, wherein the tension member is a spring.

Example 19. The method of Example 17, wherein the tension member is a shape-set V-shaped connector.

## Claims

1. A heart valve repair device having a delivery configuration and a deployed configuration, the heart valve repair device comprising:
a first anchor having an open state and a closed state;
a second anchor having an open state and a closed state; and
a tension member coupled to the first anchor and the second anchor;
wherein with the heart valve repair device in the delivery configuration, the first anchor is spaced a first longitudinal distance from the second anchor; and
wherein the tension member is configured to translate the second anchor relative to the first anchor such that the first anchor is spaced a second longitudinal distance from the second anchor, the second longitudinal distance being shorter than the first longitudinal distance.

2. The heart valve repair device of claim 1, wherein the first anchor includes a first body portion and a first claw portion, wherein the first anchor in the open state is configured to receive a first portion of a chordae therein, and wherein the first anchor in the closed state is configured to grasp the first portion of the chordae.

3. The heart valve repair device of claim 2, wherein the second anchor includes a second body portion and a second claw portion, wherein the second anchor in the open state is configured to receive a second portion of the chordae therein, and wherein the second anchor in the closed state is configured to grasp the second portion of the chordae.

4. The heart valve repair device of claim 3, wherein the tension member is configured to translate the second anchor relative to the first anchor with the chordae grasped by the first anchor and the second anchor such that the chordae is shortened.

5. The heart valve repair device of any of claims 1 to 4, wherein the first anchor and the second anchor are shape-set to the closed state.

6. The heart valve repair device of any of claims 1 to 5, wherein the tension member comprises a suture slidably coupled to the first anchor and fixedly secured to the second anchor such that pulling the tension member pulls the second anchor towards the first anchor.

7. The heart valve repair device of any one of claims 1 to 6, further comprising a lock configured to secure the tension member to the first anchor with the heart valve repair device in the deployed configuration such that the first anchor is spaced the second longitudinal distance from the second anchor.

8. The heart valve repair device of any of claims 1 to 5, wherein the tension member includes a first end fixedly secured to the first anchor and a second end fixedly secured to the second anchor, wherein the tension member is shape-set such that the first anchor is spaced the second longitudinal distance from the second anchor, and wherein in the delivery configuration, the tension member is placed in tension such that the first anchor is spaced the first longitudinal distance from the second anchor.

9. The heart valve repair device of claim 8, wherein the tension member is a spring.

10. The heart valve repair device of claim 8, wherein the tension member is a shape-set V-shaped connector.

11. The heart valve repair device of any of the preceding claims, wherein:
the heart valve repair device is configured to be delivered to a native heart valve;
the first anchor is configured to grasp a first portion of a chordae;
the second anchor is configured to grasp a second portion of the chordae such that the first anchor and the second anchor are spaced first longitudinal distance apart; and
the tension member is configure to transition to a deployed state such that the first anchor is spaced a second longitudinal distance from the second anchor, wherein the second longitudinal distance is shorter than the first longitudinal distance, thereby shortening or tightening the chordae.

12. The heart valve prosthesis of claim 11, wherein the tension member is configured to be severed after transitioning to the deployed state.
